# EUROPEAN PATENT APPLICATION

(11) **EP 2 392 342 A1**
(43) Date of publication of application: **07.12.2011**
(21) Application number: 10290300.2
(22) Date of filing: 04.06.2010
(51) Int. Cl.: A61K 38/16

(54) **Compositions for use in the treatment or diagnosis of prion diseases**

(71) Applicant: Commissariat à l'Énergie Atomique et aux Énergies Alternatives, 75015 Paris (FR)
(72) Inventor: Xu, Zhou, 75015 Paris (FR); Deslys, Jean-Philippe, 78150 Le Chesnay (FR); Rezaei, Human, 91300 Massy (FR)
(74) Representative: Vial, Lionel

(57) **Abstract**

The present invention relates to a compound comprising or consisting of poly-D-Lysine, for use in the prevention or treatment of the pathological binding of a prion protein (PrP) to a protein liable to form brain aggregates in an individual.

## Description

### Field of the invention

The present invention relates to compositions for use in prevention or treatment of the pathological binding of a prion protein (PrP) to a protein liable to form brain aggregates, and to methods for diagnosing prion diseases.

### Background of the invention

Prion diseases are a group of infectious, invariably fatal neurodegenerative disorders, also known as transmissible spongiform encephalopathies, notably comprising Creutzfeldt-Jakob disease (CJD), Gerstmann-Sträussler-Scheinker syndrome (GSS), fatal familial insomia (FFI) in humans, and bovine spongiform encephalopathy (BSE) or scrapie in animals. In particular, prion was at the origin of the well-known crisis in the 90's due to the "mad cow disease", which revealed the transmissibility of BSE to human, generating the new variant of CJD (vCJD).

Prion diseases are characterized by the conversion of a non-pathological host-encoded protein PrP^{C} into a pathological β-sheet rich isoform called PrP^{SC} or PrPres, which is resistant to proteolysis and aggregates to form filaments (Prusiner (1998) Proc. Natl. Acad. Sci. USA 95:13363-13383). The transition from PrP^{C} to PrP^{SC}, which is favored by PrP^{SC}, is a key step in the pathogenesis of prion. As such, study of the conversion of PrP^{C} and the modulation of different PrP isoforms and assemblies is considered of utmost importance for prion diseases.

In addition, Lauren et al. (2009) Nature 457:1128-1132 recently highlighted an interaction between amyloid β peptide oligomers, which might be the most relevant assemblies for Alzheimer's disease, and PrP^{C}. They further showed that PrP^{C} mediate synaptic alteration caused by the amyloid-β peptide, evidencing the role of an interaction between PrP and amyloid-β peptide in the mechanisms underlying Alzheimer's disease pathogenesis. It has been further confirmed by Gimbel et al. (2010) J Neurosci. 30:6367-74 that this interaction has relevant consequences in memory impairment associated to Alzheimer's disease.

Several therapeutic strategies have been explored for treating prion diseases, among which treatment with polycationic compounds. The use of branched polyamines, such as polyamidoamide (PAMAM) or polypropyleneimine (PPI) dendrimers has thus been proposed in this regard, as is notably reported in Supattapone et al. (1999) Proc. Natl. Acad. Sci. USA 96:14529-14534; Supattapone et al. (2001) J. Virol. 75:3453-3461; US 6,419,916; US 6,331,296; or US 2005/221404.

In addition, poly-L-lysine has been proposed to be useful for dissolving fibrils of amyloid β peptide *in vitro* in US 6,639,058 and Nguyen et al. (2002) Biochem. Biophys. Res. Commun. 291:764-768.

However, despite all these fundamental discoveries, no straightforward efficient therapy against prion diseases (Trevitt & Collinge (2006) Brain 129:2241-2265) or Alzheimer's disease is currently available.

Accordingly, it is an object of the present invention to provide novel compounds liable to yield novel treatments for prion-mediated diseases.

### Summary of the invention

The present invention arises from the unexpected finding, by the inventors, that incubation of neuronal cell lines infected by a prion strain with poly-D-lysine (PDL), but not with poly-L-lysine (PLL) or D-lysine, suppressed the presence of the pathological, proteolysis-resistant form of PrP. The inventors have further shown that this effect is mediated by a direct interaction of poly-D-Lysine with PrP^{C}, in particular in the H2H3 region of PrP^{C}, thus preventing transconformation towards the pathological form of PrP.

The present invention thus relates to a compound comprising or consisting of poly-D-Lysine, for use in the prevention or treatment of the binding, in particular the pathological binding, of a prion protein (PrP) to a protein liable to form brain aggregates in an individual.

The present invention also relates to a compound comprising or consisting of poly-D-Lysine, for use in the prevention or treatment of a transmissible spongiform encephalopathy or a prion disease in an individual.

The present invention also relates to a compound comprising or consisting of poly-D-Lysine, for use in the prevention or treatment of Alzheimer's disease, in particular by inhibiting a binding of a prion protein (PrP) to amyloid-β peptide, in an individual.

The present invention also relates to the use of a compound comprising or consisting of poly-D-Lysine for the manufacture of a medicament intended for the prevention or treatment of:
- the binding, in particular the pathological binding, of a prion protein (PrP) to a protein liable to form brain aggregates, in an individual;
   - a transmissible spongiform encephalopathy or a prion disease, in an individual; or
- Alzheimer's disease, in particular by inhibiting the binding of a prion protein (PrP) to amyloid-β peptide, in an individual.

The present invention also relates to a method for the prevention or treatment of:
- the binding, in particular the pathological binding, of a prion protein (PrP) to a protein liable to form brain aggregates;
- a transmissible spongiform encephalopathy or a prion disease; or
   - Alzheimer's disease in particular by inhibiting the binding of a prion protein (PrP) to amyloid-β peptide;
in an individual, comprising administering the individual with a prophylactically or therapeutically effective amount of a compound comprising or consisting of poly-D-Lysine.

The present invention also relates to the use of a compound comprising or consisting of poly-D-Lysine, for the *in vitro* diagnosis of a transmissible spongiform encephalopathy.

The present invention further relates to a Protein Misfolding Cyclic Amplification (PMCA) assay, comprising:
(i) adding a composition comprising PrP^{C} to a sample to assay;
(ii) incubating the sample added with the composition comprising PrP^{C} in conditions allowing the formation of PrP^{SC} aggregates in the sample;
(iii) dispersing the aggregates which may have formed in the sample;
(iv) adding a composition comprising PrP^{C} to the sample; (v) repeating steps (ii) to (iv);
(vi) determining the presence or absence of PrP^{SC} or PrPres aggregates in the sample;
   wherein the composition comprising PrP^{C} further comprises a compound comprising or consisting of poly-D-Lysine.

### Detailed description of the invention

As intended herein the prevention or treatment of the binding, in particular the pathological binding, of a prion protein (PrP) to a protein liable to form brain aggregates in an individual relates to inhibiting, *in vivo,* the formation of a complex between a prion protein (PrP), in particular PrP^{C}, with a protein liable to form brain aggregates in an individual.

A "brain aggregate" or "cerebral aggregate" according to the invention relates to any protein aggregate which may be specifically found in the brain or cerebral tissues of individual having a neurodegenerative disorder. By way of example, the brain aggregates according to the invention may be fibrils, filaments, or plaques. In addition, the protein liable to form brain aggregates according to the invention may be of any type liable to form such aggregates, however, it is preferred that the protein liable to form brain aggregates is selected from the group consisting of the prion protein, in particular PrP^{SC} or PrPres, and the amyloid-β peptide.

As intended herein the binding of the prion protein (PrP) to a protein liable to form brain aggregates is a pathological binding, in that it underlies the development of a disease. By way of example, the binding of PrP^{SC} or PrPres to PrP^{C} may cause the transconformation of PrP^{C} to PrP^{SC} or PrPres and may result in the pathological accumulation and/or aggregation of PrP^{SC} or PrPres, which is characteristic of transmissible spongiform encephalopathies or prion diseases. Also by way of example, the binding of PrP^{C} to the amyloid-β peptide, in particular in the form of amyloid-β peptide oligomers, as is notably shown by Lauren et al. (2009) Nature 457:1128-1132, may underlie particular aspects of Alzheimer's disease, such as memory loss for instance.

Preferably, the prevention or treatment, according to the invention, of the binding, in particular the pathological binding, of a prion protein (PrP) to a protein liable to form brain aggregates in an individual, is the prevention or treatment of a transmissible spongiform encephalopathy or a prion disease. In that case, as will be clear to one of skill in the art, the protein liable to form brain aggregates is a prion protein (PrP), in particular PrP^{SC} or PrPres.

Preferably also, a transmissible spongiform encephalopathy or a prion disease according to the invention is selected from the group consisting of Creutzfeldt-Jacob disease, in particular the variant of Creutzfeldt-Jacob disease (vCJD), sporadic Creutzfeldt-Jacob disease, and familial Creutzfeldt-Jacob disease; Gerstmann-Straüssler-Scheinker syndrome; fatal familial insomnia; bovine spongiform encephalopathy; and scrapie.

Preferably also, the individual may have Alzheimer's disease. In that case, as will be clear to one of skill in the art, the protein liable to form brain aggregates is the amyloid β peptide.

Without wishing to be bound to a particular theory, the inventors believe that the binding of the compound comprising or consisting of poly-D-Iysine to the prion protein, in particular PrP^{C}, prevents binding to the protein liable to form brain aggregates according to the invention. Poly-D-lysine has the property of spontaneously adsorbing to biological membranes, such as the plasma membrane, which is advantageous for crossing the Blood Brain Barrier (BBB) or hematoencephalic barrier. In addition, the blood brain barrier harbors several receptors which may increase the crossing efficiency of compounds comprising or consisting of poly-D-lysine, such as receptors for lysine or for amines, as well as members of the Multidrug Resistance-associated Protein (MRP), Solute Carrier (SLC), or Organic Anion Transporters (OAT, RST and UST) membrane protein families. Such receptors are notably described in Varatharajan & Thomas (2009) Antiviral Res. 82:A99-A109.

As intended herein, poly-D-Lysine according to the invention is a polymer of D-Lysine residues associated together through peptide bonds. The D-Lysine residues may be modified by attachment, in particular covalent attachment, of functional groups, notably intended to increase the affinity of interaction of poly-D-lysine for PrP, to decrease a potential toxicity of the compound comprising or consisting of poly-D-Iysine when administered to the individual of the invention or to improve or to provide new properties, such as crossing the blood brain barrier (BBB), targeting a specific organ (spleen for example) or targeting various metabolic/catabolic processes.

Preferably, where present, functional groups are attached to D-lysine residues on the ε-NH₂ group. Preferably also, less than 90%, 80%, 70%, 60%, 50%, 40%, 30%, 20%, or 10% of the D-Lysine residues of the poly-D-Lysine according to the invention are modified. Besides, in a particular embodiment of the invention, essentially no D-Lysine residues of the poly-D-Lysine according to the invention are modified. Preferably, poly-D-Lysine according to the invention has a molecular weight of from 4 kDa to 300 kDa, more preferably of from 15 kDa or 30 kDa to 150 kDa, and most preferably of from 70 kDa to 150 kDa.

In a particular embodiment of the invention, the compound comprising poly-D-Lysine according to the invention may further comprise other groups linked to poly-D-Lysine, preferably attached, in particular covalently attached, to the N-terminus or to the C-terminus of poly-D-Lysine. By way of example, said groups may be peptides, polypeptides, proteins, such as monoclonal antibodies, carbohydrates, lipids, such as phospholipids and glycerides, fatty acids, or sulfate groups.

As will be clear to one of skill in the art, the covalent attachment of functional groups or groups as defined above, to poly-D-lysine according to the invention may notably proceed through linker moieties. Biotine is a particularly preferred linker moiety in the frame of the present invention.

In another particular embodiment of the invention, the compound comprising or consisting of poly-D-lysine according to the invention only consists of poly-D-Lysine according to the invention.

The individual according to the invention is preferably a mammal, more preferably a human.

Administration of the compound comprising or consisting of poly-D-lysine according to the invention to the individual may proceed through any route suitable for delivering poly-D-lysine to cerebral tissues of the individual, however, the intravenous route, the intrathecal route, the inhalational route or the oral route are preferred, and the oral route is particularly preferred. In particular, where administration routes other than the intrathecal route are to be used, the compound comprising or consisting of poly-D-Lysine according to the invention may be associated to vectors intended for crossing the blood brain barrier. These vectors intended for crossing the blood brain barrier may be groups or functional groups attached to poly-D-Lysine as defined above. Alternatively, the vectors may be pharmaceutically acceptable carriers non-covalently associated to the compounds comprising or consisting of poly-D-Lysine according to the invention. Vectors intended for crossing the blood brain barrier according to the invention are well-known to one of skill in the art and are preferably selected from the group consisting of polyethylene glycol (PEG), liposomes, in particular magnetic liposomes such as described by Saiyed et al. (2010) Int J Nanomedicine 5:157-166, fatty acids, glycerides, phospholipids, nanoparticles or monoclonal antibodies.

PMCA is a method well-known to one of skill in the art, first reported in Saborio et al. (2001) Nature 411:810-3. It is notably described by Deleault et al. (2007) Proc. Natl. Acad. Sci. USA 104:9741-9746 and by Grassi et al. (2008) Vet. Res. 39:33. Advantageously, adding a compound comprising or consisting of poly-D-Lysine according to the invention to a composition comprising PrP^{C} to be used in a Protein Misfolding Cyclic Amplification (PMCA) assay is useful for preventing spontaneous generation of PrP^{SC} or PrPres from PrP^{C}, which may occur in this method and which may be the cause for a loss of specificity of the method.

Preferably, the PMCA assay according to the invention is for use in the *in vitro* diagnosis of a transmissible spongiform encephalopathy or a prion disease. Preferably also, in the PMCA assay according to the invention the sample to assay is biological sample, more preferably a biological sample selected from the group consisting of a blood sample or a cerebrospinal fluid sample and most preferably a blood sample, such as a whole blood sample, a plasma sample and a serum sample.

### Description of the figures

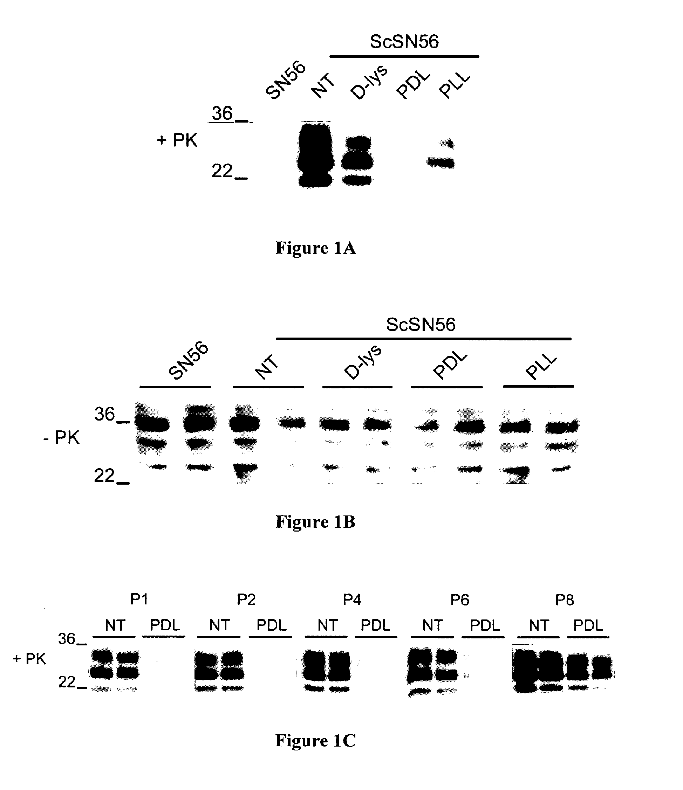

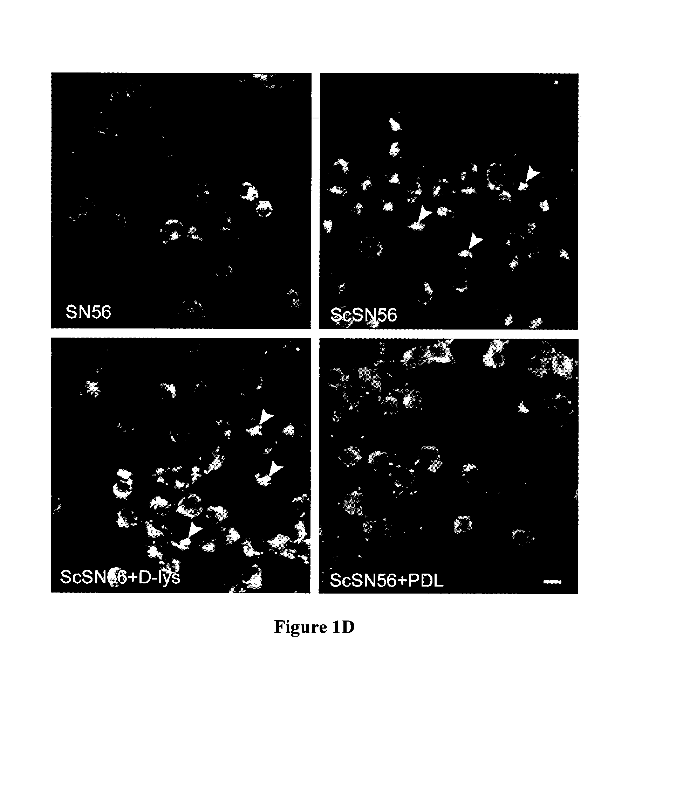

- Figures 1A, 1B, 1C and 1D: Elimination of PrPres by PDL in ScSN56.
   Figures 1A and 1B represent a Western blot analysis using anti-PrP monoclonal antibody Saf83: on uninfected SN56, Chandler-infected ScSN56 treated with D-lysine (D-lys), PDL 70-150 kDa (PDL), PLL 70-150 kDa (PLL) or not treated (NT). In figure 1A, Proteinase K (PK)-resistant PrP is removed by PDL treatment. In Figure 1B, total PrP, without PK digestion, does not change in a major way either in its amount or in its glycosylation pattern. All conditions are duplicated.
   Figure 1C represents a Western blot of PK-digested duplicate samples over several passages (P1 to P8).
   Figure 1D represents the immunofluorescent labelling and microscopy visualization of permeabilized SN56 and ScSN56 treated with D-lysine (lower left), PDL (lower right) or not treated (upper right). White arrows point to morphologically ScSN56-specific aggregates. Scale bar, 20 µm.
- Figures 2A, 2B and 2C: PDL interacts with H2H3 domain of PrP without change of secondary structure.

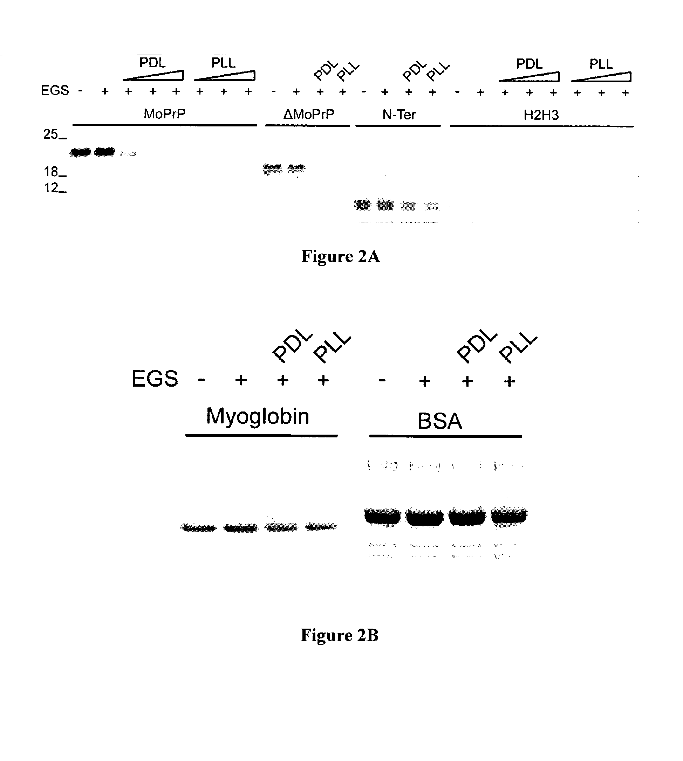

   Figures 2A and 2B represent ethylene glycol bis-succinimidyl succinate (EGS) cross-linking assay. Target proteins at 20 µM include (Figure 2A) full-length mouse recombinant PrP (MoPrP) and truncated mutants PrP99-230 (ΔMoPrP), PrP23-124 (N-Ter), PrP170-230 (H2H3) as well as controls (Figure 2B) myoglobin and bovine serum albumin (BSA). Each protein is shown either alone or incubated only with EGS or with EGS and PDL/PLL (20 µM). For MoPrP and H2H3, three concentrations (10, 20 and 40 µM) of PDL/PLL are tested.

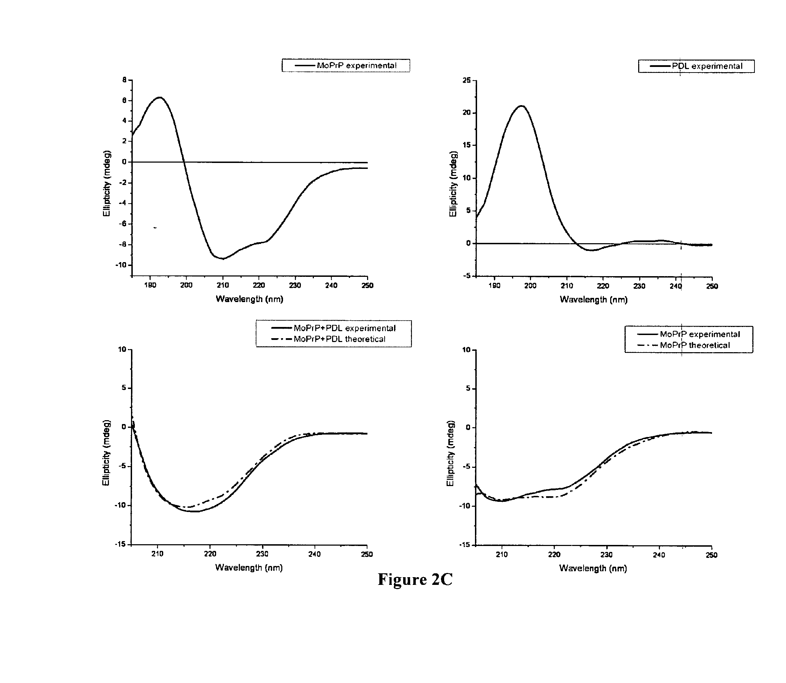

   Figures 2C represent a CD analysis showing that PrP secondary structure is not altered in the presence of PDL. Upper left panel represents a far-UV spectrum of MoPrP (50 µM). Upper right one represents PDL (50 µM) spectrum. Lower left panel represents the spectrum of the equimolar mixture (experimental, continuous line) superimposed with the algebraic sum of the MoPrP and PDL spectra (theoretical, dotted line). Lower right panel represents a processed spectrum of the equimolar mixture of MoPrP and PDL (50 µM each) with the algebraic subtraction of the PDL spectrum (theoretical, continuous line), superimposed with the experimental spectrum of MoPrP (50 µM) (experimental, dotted line).

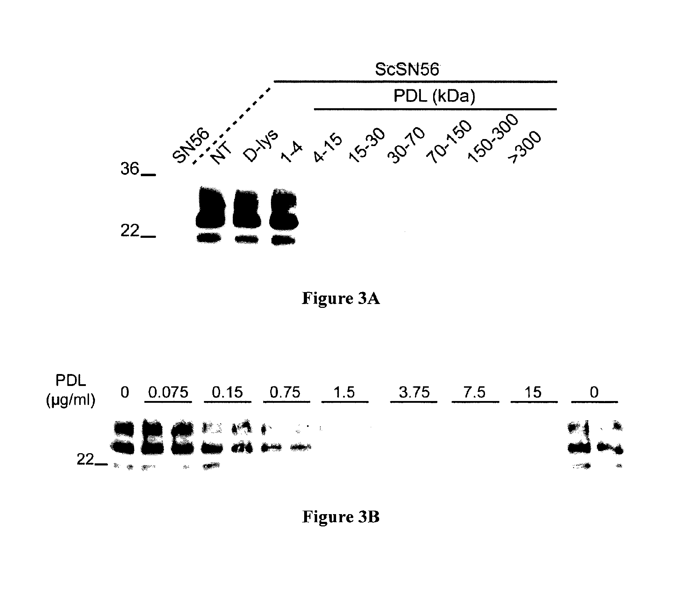

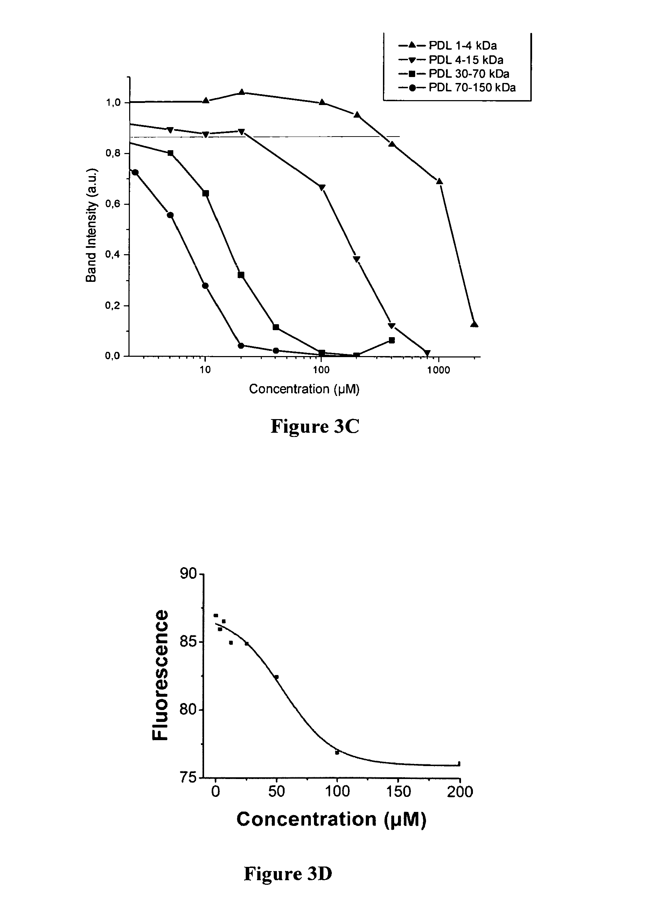

- Figures 3A, 3B, 3C and 3D: PDL effect on prion is size and concentration-dependent
   Figures 3A and 3B represent western blots of PrPres from ScSN56 culture treated with different sizes of PDL, using the concentration 7.5 µg/ml in the medium (Figure 3A) and increasing concentrations, using PDL 70-150 kDa (Figure 3B).
   Figure 3C represents a densitometric analysis of Coomassie-stained SDS-PAGE signals from EGS cross-linking assays using ranges of concentration and four sizes of PDL: 1-4 kDa (up-sided triangle), 4-15 kDa (down-sided triangle), 30-70 kDa (square) and 70-150 kDa (circle).
   Figure 3D represents a Fluorescence-quenching assay using ΔOvPrP-1206W and increasing concentrations of PDL 70-150 kDa.

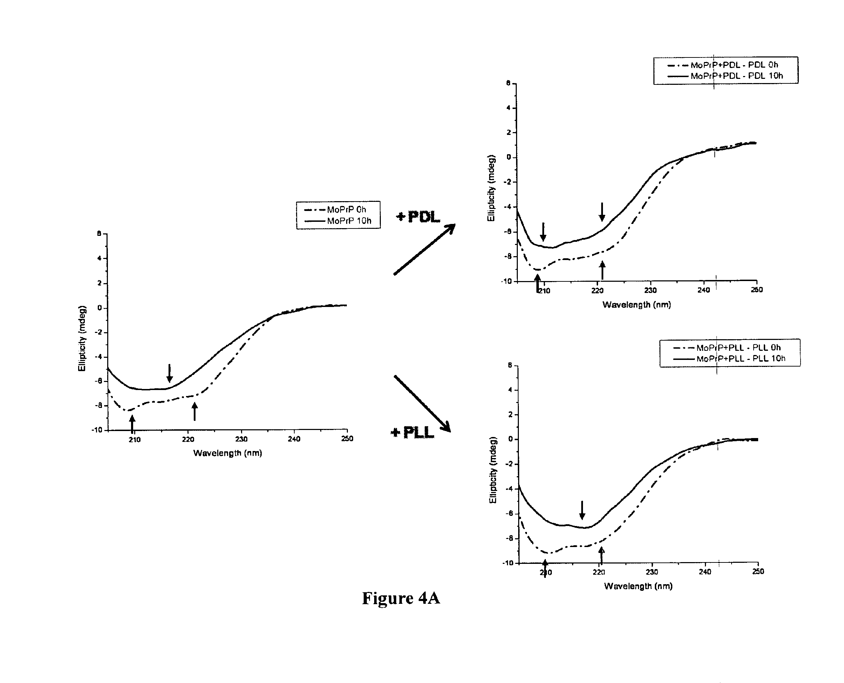

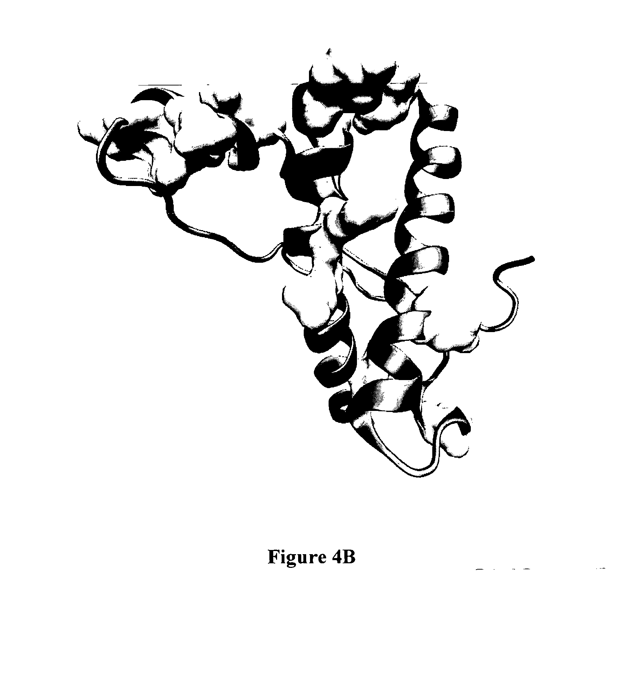

- Figures 4A and 4B: Enantiospecific inhibition of MoPrP α to β conversion by PDL
   Figures 4A represents MoPrP at 50 µM was incubated for 10 hours at 57°C either alone (left panel), with PDL (50 µM) (upper right panel) or with PLL (50 µM) (lower right panel). For incubation with PDL and PLL, spectra are processed by subtracting the spectrum of PDL or PLL from the spectra of the mixture, so that only the MoPrP component is shown. Displayed spectra are the first (dotted line and the last (normal line) of the kinetics.
   Figure 4B represents the NMR structure of MoPrP where negatively charged residues are shown in white (globular side chains).

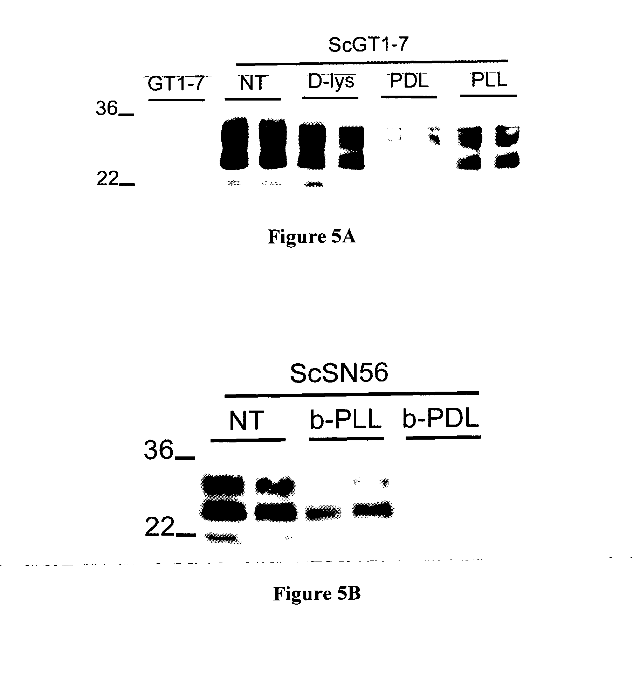

- Figures 5A and 5B: PDL treatment of infected GT1-7 and binding of PDL on cells
   Figure 5A represents Western blot analysis of PrPres in GT1-7, which reproduces the effect of PDL observed in SN56 cell line. Western blot using Saf83 shows uninfected GT1-7 cells (GT1-7), 22L-scrapie-infected GT1-7 (ScGT1-7) treated with D-lysine (D-lys), PDL 70-150 kDa (PDL), PLL 70-150 kDa (PLL) or not treated (NT). All conditions are duplicated.
   Figure 5B represents the quantity of PDL or PLL bound on ScSN56 surface. After 24 hours, more PDL is bound on ScSN56 surface than PLL. Figure 5B shows that biotinylation of PDL and PLL does not affect the results observed without this modification. Western blot analysis of PrPres in ScSN56 treated with biotinylated PDL (b-PDL), biotinylated PLL (b-PLL) or not treated (NT). Biotinylation of PDL/PLL was performed using EZ-Link Biotinylation kit (Pierce) at the ratio ∼22 biotins/molecule.

The invention will be further illustrated by the following nonlimiting Examples.

### EXAMPLES

### Materials and methods

### Materials

Poly-D-lysine (PDL) hydrobromide of various sizes (1 to 4, 4 to 15, 15 to 30, 30 to 70, 70 to 150, 150 to 300, or >300 kDa), poly-L-lysine (PLL) hydrobromide, D-lysine, ethylene glycol bis-succinimidyl succinate (EGS) were purchased from Sigma-Aldrich. Unless specified otherwise, the size of PDL used in the experiments was 70-150 kDa.

### Recombinant PrP Purification.

The full-length recombinant mouse PrP and deletion mutants were produced and purified as described in Rezaei et al. (2000) Eur. J Biochem. 267:2833-2839. Briefly, genes encoding full-length mouse PrP (PrP23-230), C-terminal mutant (PrP 99-230), N-terminal mutant (PrP23-124) and H2H3 mutant (PrP170-230), notably described by Chakroun et al., (2010) FASEB J. 24:1-10, were cloned into *Escherichia coli* and expressed. Inclusion bodies were extracted, sonicated and solubilised. Purification was performed on a Ni Sepharose column (GE Healthcare) using the high affinity between metal cations and the N-terminal part of the prion protein or 6xHis tag for mutants lacking the N-terminal part.

### Cell Culture

The mouse cholinergic septal neuronal cell line SN56 described in Magalhaes et al. (2005) J Neurosci. 25(21):5207-5216 was grown in a culture medium composed of OptiMem (Gibco), supplemented with 10% foetal calf serum (FCS) and 1% Penicillin-Streptomycin (PS). The hypothalamic neuronal GT1-7 cell line described in Schätzl et al. (1997) J Virol. 71:8821-8831 was maintained in OptiMem, with 5% FCS, 5% horse serum and 1% PS. Cells were inoculated by clarified homogenates from brains of mice infected by Chandler or 22L Prion strains, as described in Vilette et al. (2001) PNAS 98:4055-4059, and then chronically maintained.

Unless stated otherwise, incubation with PDL, PLL or other compounds was done on the third day after passage, for 24 hours in culture medium, prior to extensive rinse with PBS then replating and/or analysis. A PDL concentration-dependence experiment was carried out and on the basis of the result **(****Fig. 3B****)**. The 7.5 µg/ml concentration was thus validated in culture medium for all compounds for standard use in all cell culture experiments.

### Immunofluorescent Labelling and Microscopy.

Cells were grown in Lab-Tek slides (Nunc) following the cell culture procedure. For immunofluorescent labelling of PrP, cells were fixed with 4% paraformaldehyde, permeabilized with 0.1% Triton and treated with 4.23 M guanidium thiocyanate. The Saf83 monoclonal antibody was used to detect PrP and was revealed with Alexa Fluor 488-conjugated secondary antibody (Molecular Probes). Cover slips were mounted on slides using the Vectashield Hardset mounting medium with DAPI (Vector Labs). Image acquisition was performed on a Leica DM6000B fluorescence microscope.

### Western Blot Analysis of PrPres from Cell Culture.

After incubation with or without PDL, PLL or other indicated compounds, cells were trypsinized, and lysed for 10 min at 4°C. Nuclei and cellular remnants were removed by centrifugation at 10⁴ g for 2 min. The amount of protein was determined and normalized to 200 µg using bicinchoninic acid protein assay (Uptima). Samples were treated by proteinase K (PK) for 30 min at 37 °C, phenylmethylsulfonyl fluoride (PMSF 1 mM) was added to stop digestion and proteins were centrifuged at 1.5x10⁴ g for 1 h at 4°C. The pellets were resuspended and heated for 5 min at 100°C in 25 µl of Laemmli buffer. For analysis of total PrP, lysates (7.5 µg of total protein) were not PK-digested but directly diluted in 2x Laemmli buffer.

Samples were subjected to sodium dodecyl sulfate 12% polyacrylamide gel electrophoresis (SDS-PAGE) and electroblotted to nitrocellulose membranes in transfer buffer (12 mM Tris; 80 mM glycine; 10% isopropanol), at 20 V overnight. The membranes were blocked in 5% (wt/vol) fat-free milk, processed with the Saf83 antibody and an anti-mouse horseradish-peroxidase coupled secondary antibody. Revelation was performed using the ECL kit (Amersham Pharmacia Biotech) by an autoradiographic method.

### Spectropolarimetry and Spectrofluorimetry.

Circular Dichroism (CD) experiments were performed in a Jasco 810 device with a home made thermostatic apparatus adapted to a 0.01 cm circular quartz cuvette. Recombinant full-length PrP was used at 50 µM in 5 mM MOPS buffer adjusted to pH 7.0, with 100 mM NaF, with or without PDL or PLL at 50 µM. For analysis of structural change upon interaction, spectra were recorded at 20°C. For the study of α to β transition kinetics of PrP, spectra were recorded at 57°C every 10 minutes, over 10 hours, using a 0.1 nm data pitch and a 20 nm.min⁻¹ scanning speed. The wavelength scale is adapted to the saturation of the photomultiplicator.

For the fluorescence-quenching assay, ovine truncated (aa 103-232) 1206W mutant recPrP (ΔOvPrP-1206W) was used at 50 µM in 50 mM MOPS buffer adjusted to pH 7.0. Increasing concentrations (0 to 200 µM final concentrations) of PDL 70-150 kDa were added to the solution. The single tryptophan fluorescence was recorded (excitation at 280 nm, emission spectrum from 295 to 450 nm) in a Jasco FP-6200 spectrofluorimeter and the evolution of the maximum at 348 nm of the spectrum was plotted.

### EGS Cross-linking Experiment.

Unless stated otherwise, cross-linking was induced at 4°C by the addition of EGS (20 µM final concentration) in a solution of 20 µM recombinant full-length mouse PrP, mutants or other proteins with or without 20 µM PDL or PLL in 50 mM MOPS buffer at pH 7.0. Reactions were stopped after 10 min by an excess of amine groups with 15% Tris-glycine buffer. After addition of an equal volume of 2x Laemmli buffer, samples were heated for 5 min at 100°C and then subjected to SDS-PAGE in a 12% polyacrylamide gel. Staining of gels with Coomassie blue followed standard procedure.

### Example 1: Poly-D-lysine, but not D-lysine nor poly-L-lysine, effectively removes PK-resistant PrP from infected neuroblastoma cells

SN56 cells chronically infected with Chandler scrapie prion strain (hereafter referred to as ScSN56), were studied in the presence and absence of PDL (70-150 kDa), D-lysine or PLL (70-150 kDa).

The amount of PrPres was analyzed after proteinase K digestion of cell lysates by Western blots with the PrP-specific monoclonal antibody Saf83.

Incubation of ScSN56 with 7.5 µg/ml PDL for 24 hours in the medium was sufficient to decrease the signal of proteinase-K-resistant PrP down to trace amount, **(****Fig: 1A****).** No cytotoxic effect was observed with this transient exposure. However, in these conditions, only a faint reduction of PrPres could be detected with PLL, as previously described in Supattapone et al. (1999) PNAS 96:14529-14534, and no effect with D-lysine **(****Fig.1A****).**

Similar observations were made in another cell line GT1-7, which was infected with the 22L scrapie prion strain **(****Fig. 5A****)**.

Therefore the removal of PrPres in two different cell lines, infected by two different prion isolates, was specific for poly-D-lysine.

Although after treatment, cells were carefully washed in PBS to remove PDL, a putative interference of the remaining PDL with subsequent purification process including PK-digestion could not be completely excluded. To circumvent this uncertainty, a single treatment with PDL was done at passage 0 and the presence of PrPres was assessed after 1, 2, 4, 6 and 8 passages **(****Fig. 1C****).** Wash steps and 1:10 dilution replating of cells at each passage reduced the PDL left on the culture down to a negligible amount. **Fig. 1C** showed that, up to passage 6, only trace amount of PrPres was detected without significant increase compared to passage 1. A strong PrPres signal reappeared at passage 8. Thus, discarding the possibility of an artefact, this experiment demonstrated that the infected cells were effectively and stably cured for several passages, after a single 24-hour treatment.

The observation of PDL effect on PrPres was also supported by immunofluorescent labelling of PrP. Microscopy analysis showed the presence of a specific intracellular aggregate, beside the membrane labelling, in infected cells (ScSN56), as well as in D-lysine-treated ones, that is different from the labelling of uninfected SN56, supposed to be essentially on the membrane, in the Golgi apparatus and endoplasmic reticulum as reported previously (Kristiansen et al. (2005) J Biol. Chem. 280:38851-38861; review Harris (2003) British Medical Bulletin 66:71-85; review Westergard et al. (2007) Biochim Biophys Acta 1772(6):629-644). Incubation with PDL, but not D-lysine, purged ScSN56 of this specific aggregate making them undistinguishable from uninfected cells.

To determine whether this enantiospecific removal of PrPres by PDL was due to a decrease in either expressed PrP^{C} or a modification of membrane localization, the amount of total PrP and surface PrP was evaluated by Western blot and flow cytometry respectively. The estimation by Western blot of total PrP in cell lysate revealed no significant variation between controls and treated samples **(****Fig. 1B****).** This figure also showed no obvious difference in glycosylation pattern. This lack of total PrP variation has to be compared to the nearly complete disappearance of PrPres signal reported in **Fig. 1A** **and** **5A****.**

### Example 2: Poly-n-lysine interacts with the H2H3 region of PrP.

To determine if the effect of PDL involves a direct link with PrP, the inventors chose to probe a potential physical interaction by cross-linking mouse recombinant PrP (MoPrP) and PDL with ethylene glycol bis-succinimidyl succinate (EGS), a 16 A linear molecule that creates covalent links between two sufficiently close amine groups.

Full-length MoPrP, truncated mutants PrP99-230 (ΔMoPrP), PrP23-124 (N-Ter) and PrP170-230 (H2H3) were incubated on ice for 10 minutes with or without PDL or PLL in the presence of EGS **(****Fig. 2A****)**. It should be noted that at pH < 11, polylysines are positively-charged large molecules that cannot enter standard SDS-PAGE gels which results in the blockade of the entry to the gel of any protein cross-linked to PDL or PLL.

As shown in **Fig. 2A**, a decrease of the Coomassie blue staining of MoPrP in the presence of PDL was observed, indicating an interaction between the two molecules **(****Fig. 2A****, lanes "MoPrP")**. No spontaneous dimerization or oligomerization of MoPrP incubated alone with EGS could be seen saw and no sign of interaction between polylysines and control proteins, such as bovine serum albumin and myoglobin, could be detected **(****Fig. 2B****)**. Interaction with PLL showed no difference compared to PDL.

To precisely localize the region involved in PrP/PDL interaction, deletion mutants of PrP were used. As shown in **Fig. 2A**, the N-terminal region (N-Ter) did not interact with PDL, whereas the C-terminal region (ΔMoPrP) and particularly H2H3 interacted with PDL.

Since oligomerization and fibrillization of PrP require a conformational change, the inventors wondered whether the interaction of PrP with PDL or PLL would affect its secondary structure.

Far-UV circular dichroism (CD) spectra were recorded for monomeric MoPrP **(****Fig. 2C****, upper left,** PDL **(****Fig. 2C****, upper right)** and their equimolar mixture.

The inventors reasoned that if there were a change in secondary structure either in MoPrP or PDL upon interaction, the algebraic subtraction of PDL spectrum from the experimental spectrum of the mixture would not fit the original individual spectrum of MoPrP. Given the resolution of CD, no fundamental difference between them could be observed **(****Fig. 2C****, lower right)**, indicating that the interaction between MoPrP and PDL involved no major change in the secondary structure of MoPrP monomers.

Because PrP oligomers display a different structure, enriched in β-sheet, compared to monomeric PrP, the inventors also tested whether PDL and PLL interacted with heat-oligomerized MoPrP.

EGS cross-linking assay showed that PDL and PLL interacted with heat-oligomerized MoPrP similarly to native MoPrP monomer. Likewise, using circular dichroism, the effect of PDL on the secondary structure of oligomerized MoPrP was evaluated. The subtraction of the spectrum of PDL from that of the mixture led to a curve that is typical of a β-sheet structure, also displayed by the oligomerized MoPrP control.

Overall, polylysines directly interacted with the H2H3 region of MoPrP monomer and oligomer, without affecting its secondary structure in a detectable way.

### Example 3: PrPres removal in cell culture and interaction in vitro with poly-D-lysine are both concentration- and size-dependent.

To further characterize both the effect on cell culture and the *interaction in vitro,* PDL concentration and size-dependence were studied. In infected cells, increasing PDL concentration in the medium enhanced the efficiency of PrPres removal, giving an approximation of the concentration of PDL needed to lower the amount of PrPres by 50 %, which is around 0.15 µg/ml, corresponding to the nanomolar range **(****Fig. 3B****).**

The influence of the size of the polymers on the removal of PrPres was then probed. While incubation with D-lysine and 1-4 kDa PDL did not result in significant change in signal compared to untreated control, a similar level of removal of PrPres for all other sizes of PDL, from 4-15 kDa up to over 300 kDa could be observed **(****Fig. 3A****)**.

Similarly, interaction probed by EGS cross-linking showed that interaction was concentration-dependent and that large polymers had a higher propensity to interact with MoPrP **(****Fig. 3C****).**

As cross-linking experiments are not suitable for providing numerical affinity or avidity estimates, the presence of EGS being a bias in the reaction equilibrium, a fluorescence-quenching assay was performed. Based on the result that the interaction sites may be on H2H3 **(****Fig. 2A****),** a truncated mutant of ovine PrP (aa 103-232, which then displayed no tryptophan) was generated on which the isoleucine residue at position 206 on H3 was replaced by a tryptophan (ΔOvPrP-I206W). The titration of ΔOvPrP-1206W by PDL revealed a decrease of the single tryptophan fluorescence indicating a modification of H3 environment. The concentration of PDL (70-150 kDa) required to cross-link half of the MoPrP monomers in the EGS assay was within the range of concentration needed to decrease the fluorescence by half in the fluorescence-quenching experiment **(****Fig. 3D****)**, which is in the order of magnitude of 10 µM.

### Example 4: Poly-D-lysine, but not poly-L-lysine, prevents heat-induced a to β transition of PrP.

Change in conformation of PrP is a key step in the formation of prion (Caughey et al. (1991) Biochem. 30:7673-7680; Pan et al. (1993) PNAS 90:10962-10966). The inventors thus probed by CD whether PDL or PLL could influence the heat-induced transition of MoPrP from an α-helix-rich structure to a β-sheet one.

Results in **Fig. 2C**, lower right panel, showed that interaction of MoPrP with polylysines did not induce *per se* any major change in secondary structure of MoPrP. In other words, from a practical point of view, it was possible to retrieve the CD spectrum of MoPrP by subtracting that of polylysine from the spectrum of the mixture. The same reasoning was applied in the study of α to β transition of MoPrP at pH 7.0 at 57°C over 10 hours, in the presence or absence of polylysine.

When incubated alone, the spectrum of PDL over time displayed no significant change of structure. In the case of MoPrP heated alone, results showed a typical transition from a high α-helix content to a high β-sheet content **(****Fig. 4A****, left panel),** characterized by the disappearance of local minima after 10 hours.

In the spectra of the equimolar mixture of MoPrP and PDL (respectively PLL), the spectrum of PDL (resp. PLL) was subtracted to specifically visualize the MoPrP component. In the presence of PDL, the processed spectrum was maintained over time and constantly corresponded to an α-helix-rich structure **(****Fig. 4A****, upper right panel)**, whereas the presence of PLL allowed a visible change from α to β **(****Fig. 4A****, lower right panel)** similar to the transition in the spectrum of MoPrP incubated alone **(****Fig. 4A****, left panel).**

Overall, the results demonstrated an enantiospecific effect of PDL on the heat-induced α to β transition of MoPrP, maintaining it in an α-helix-rich structure.

In conclusion, it is herein reported for the first time that PDL has an anti-prion effect. Indeed, PDL drastically removes PrPres from prion-infected neuronal cell culture without affecting significantly the expression and the membrane localization of PrP^{C}. Furthermore, through *in vitro* investigations a physical interaction was identified between PrP and PDL. This interaction provides the key to hypothesize that PDL acts on prion by preventing the conversion of normal cellular PrP^{C} into its abnormal β-sheet-enriched isoform PrP^{SC}. This mechanism is strengthened by the fact that PDL physically interacts with the H2H3 domain, which is implicated in the oligomerization and fibrillization of the full-length protein. Consequently, this report strongly supports the hypothesis that H2H3 may be the most important region of PrP for abnormal conversion and polymerization, as it would bridge the gap between biophysical and modelling studies on H2H3 and biological relevance. Evidence provided here implies that drugs, rationally designed to target H2H3 on PrP^{C}, can block prion replication. Therefore, new therapeutic strategies based on H2H3 find here fundamental support and consistence.

## Claims

1. A compound comprising or consisting of poly-D-Lysine, for use in the prevention or treatment of a pathological binding of a prion protein (PrP) to a protein liable to form brain aggregates in an individual.

2. The compound for use according to claim 1, wherein the protein liable to form brain aggregates is selected from the group consisting of the prion protein and the amyloid-β peptide.

3. The compound for use according to claim 1 or 2, in the prevention or treatment of a transmissible spongiform encephalopathy.

4. The compound for use according to any of claims 1 to 3, in the prevention or treatment of a transmissible spongiform encephalopathy selected from the group consisting of Creutzfeldt-Jacob disease, Gerstmann-Straüssler-Scheinker syndrome, fatal familial insomnia, bovine spongiform encephalopathy, and scrapie.

5. The compound for use according to any of claims 1 to 4, in the prevention or treatment of Creutzfeldt-Jacob disease, the variant of Creutzfeldt-Jacob disease, sporadic Creutzfeldt-Jacob disease, and familial Creutzfeldt-Jacob disease.

6. The compound for use according to claim 1 or 2, wherein the individual has Alzheimer's disease.

7. The compound for use according to any of claims 1 to 6, wherein poly-D-Lysine has a molecular weight of from 4 kDa to 300 kDa.

8. The compound for use according to any of claims 1 to 7, wherein poly-D-Lysine has a molecular weight of from 70 to 150 kDa.

9. The compound for use according to any of claims 1 to 8, wherein the compound consists of poly-D-Lysine.

10. Use of a compound comprising or consisting of poly-D-Lysine, for the *in vitro* diagnosis of a transmissible spongiform encephalopathy.

11. The use according to claim 10, wherein the compound comprising or consisting of poly-D-Lysine is as defined in any of claims 7 to 9.

12. A Protein Misfolding Cyclic Amplification (PMCA) assay, comprising:
(i) adding a composition comprising PrP^{C} to a sample to assay;
(ii) incubating the sample added with the composition comprising PrP^{C} in conditions allowing the formation of PrP^{SC} aggregates in the sample;
(iii) dispersing the aggregates which may have formed in the sample;
(iv) adding a composition comprising PrP^{C} to the sample;
(v) repeating steps (ii) to (iv);
(vi) determining the presence or absence of PrP^{SC} or PrPres aggregates in the sample;
wherein the composition comprising PrP^{C} further comprises a compound comprising or consisting of poly-D-Lysine.

13. The PMCA assay according to claim 12, wherein the compound comprising or consisting of poly-D-Lysine is as defined in any of claims 7 to 9.

14. The PMCA assay according to claim 12 or 13, for the *in vitro* diagnosis of a transmissible spongiform encephalopathy.

15. The PMCA assay according to any of claims 12 to 14, wherein the sample to assay is a blood sample.
